# EUROPEAN PATENT SPECIFICATION

(11) **EP 0 861 906 B1**
(45) Date of publication and mention of the grant of the patent: **01.03.2006**
(21) Application number: 98301581.9
(22) Date of filing: 02.03.1998
(51) Int. Cl.: C12Q 1/68

(54) **Fluorescence energy transfer by competitive hybridization**
Fluoreszens-Energie-Transfer durch kompetitive Hybridisierung
Transfert d'énergie de fluorescence par hybridisation compétif

(30) Priority: 28.02.1997 US 39583 P
(43) Date of publication of application: 02.09.1998
(73) Proprietor: Quest Diagnostics Investments Incorporated, Wilmington, DE 19899 (US)
(72) Inventor: Chiang, Chih-Sheng, Vannuys, California (US); Cuan, Jose F., Vannuys, California (US)
(74) Representative: Kühn, Armin

(56) References cited:
- US-A- 5 538 848
- US-A- 5 691 145
- US-A- 5 691 146

## Description

### Scope of the Invention

This invention relates to unequal length complementary probes which have a fluorophore on one probe and a quencher on the other. The fluorophore and quencher are juxtaposed in a manner such that the proximity of the quencher to the fluorophore quenches the fluorescence of the fluorphore. These probes are useful in detecting nucleotides with sequence complemenarity. The detection capabilities reside in the competitive hybridization resulting from the use of unequal length probes, and the subsequent decrease in quenching brought about by this competitive hybridization.

### Area of the Invention

Nucleic acid amplification techniques have added to the collection of techniques by which very small quantities of a nucleotides can be enhanced to a concentration where they can be detected by some means. Several amplification techniques have become available. The most wide-spread technique is that of polymerase chain reaction, or PCR as it is now commonly called. While the amplification techniques increase the number of target nucleotide sequences available for detection, or recovery and use, a sensitive method is needed to detect the amplification product. Also, amplification technologies benefit from real-time monitoring of the amplification process. Real-time monitoring can detect non-reactive amplification runs, or detect inefficiencies in the process. Quantification of oligonucleotide burden may also be possible with real-time monitoring if such monitoring can be done without interfering with the amplification reaction.

The procedure of this invention is based on the fluorescence energy transfer between a fluorophore labeled probe and a quencher labeled probe, with sequence complementarity to each other. The probes used are of unequal length favoring the annealing of one probe to the target nucleic acid sequence over annealing to its complementary probe. In the absence of nucleic acids with sequences complementary or identical to the probe sequences (target sequence), the two probes would anneal to each other. When the two probes are annealed to each other, the proximity of the quencher to the fluorophore produces quenching of the fluorescence of the fluorophore. In the presence of nucleic acid with sequences complementary or identical to the probe sequence, some of the fluorophore labeled probe will hybridize to the nucleic acid with the complementary sequence and be separated from the quencher and yield increased (unquenched) fluorescence. This difference in fluorescence can be used for specific detection of the presence of nucleic acids with the target sequences.

### Summary of the Invention

In a first aspect, this invention relates to a method for monitoring nucleic acid amplification comprising:performing nucleic acid amplification on a target polynucleotide wherein the amplification is carried out using any method using a first oligonucleotide probe and a second shorter oligonucleotide probe varying in length by at least about 2 base pairs; the first probe having a fluorophore;the second being complementary with the first probe and having a quencher molecule capable of quenching the fluorescence of said fluorophore, the fluorophore and quencher being attached on their respective probes at positions which results in the quencher molecule quenching the fluorescence of the fluorophore when the probes are hybridized, wherein the longer probe binds preferentially to the target polynucleotide and when preferentially bound to the target polynucleotide the fluorescence intensity of the fluorophore is greater than the fluorescence intensity of the fluorophore when hybridized to the second probe, and monitoring the fluorescence of the flurorphore, the generation of fluorescence corresponding to the occurrence of nucleic acid amplification.

It also relates to a method for detecting the presence of a target polynucleotide using the unequal length probes.

The annealed probes with fluorophore and quencher are also part of this invention.

### Brief Description of the Drawings

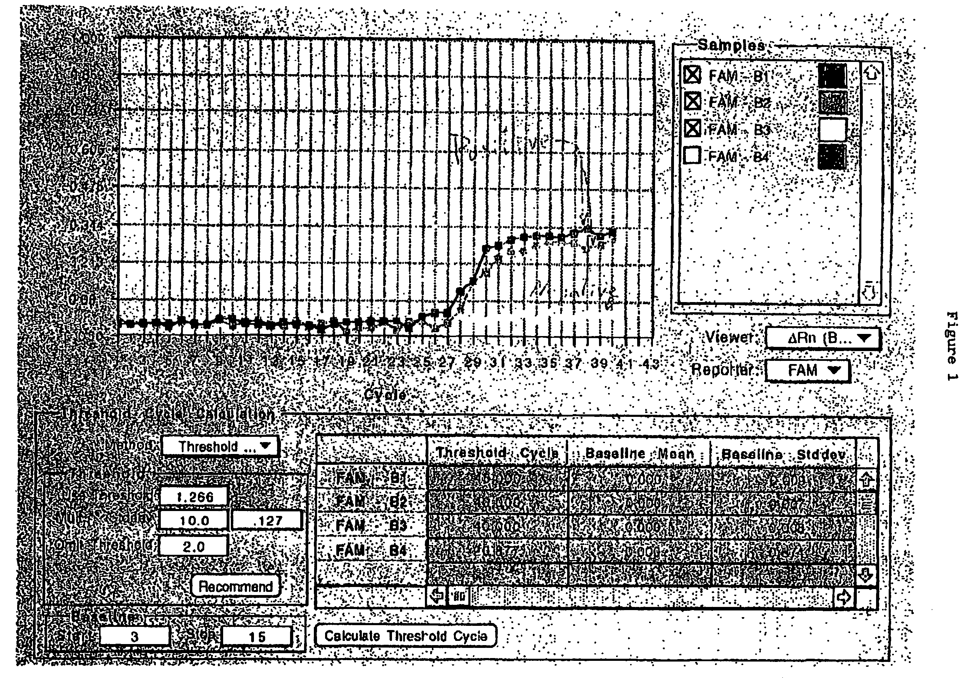

Fig 1 illustrates the fluorescence signal obtained from a PCR amplification of target HCV RNA using unequal length probes where the longer probe had fluorophore on the 5' terminal carbon, the shorter probe had a quencher on the 3' terminal carbon and was prepared by deleting three base pairs from the 5' terminus of the longer probe.

### Detailed Description of the Invention

This invention is used in conjunction_with the amplification of a target polynucleotide by by any method. These amplification techniques include PCR, ligase chain reaction (LCR), gap LCR, transcription mediated amplification (TAM), nucleic acid sequence based amplification (NASBA), and strand displacement amplification (SDA).

PCR is of greatest interest. PCR is described in many references, such as Innis et at, editors, PCR Protocols (Academic Press, New York, 1989); Sambrook et at, Molecular Cloning, Second Edition (Cold Spring Harbor Laboratory, New York, 1989); and the like. The binding site of the oligonucleotide probe is located between the PCR primers used to amplify the target polynucleotide. The PCR may be carried out using any polymerase. Because the intensity of the fluorescence signal intensifies as more replicates are made of the target polynucleotide, any polymerase which increased the number of target polynucleotides will work in this method. Preferably, PCR is carried out using a thermostable polymerase. The preferred enzyme is a Taq DNA polymerase, e.g. Amplitaq™. (Perkin-Elmer, Norwalk, Conn.), or an equivalent thermostable DNA polymerase. The annealing temperature of the PCR will be about 5 degree - 10 degree C. below the melting temperature of the oligonucleotide probes employed. The polymerase Pwo has also been use with success in this invention.

The term "oligonucleotide" as used herein includes linear oligomers of natural or modified monomers or linkages, including deoxyribonucleosides, ribonucleosides, and the like, capable of specifically binding to a target polynucleotide by way of a regular pattern of monomer-to-monomer interactions, such as Watson-Crick type of base pairing, or the like. Usually monomers are linked by phosphodiester bonds or analogs thereof to form oligonucleotides ranging in size from a few monomeric units, e.g. 3-4, to several tens of monomeric units. Whenever an oligonucleotide is represented by a sequence of letters, such as "ATGCCTG," it will be understood that the nucleotides are in 5' fwdarw 3' order from left to right and that "A" denotes deoxyadenosine, "C" denotes deoxycytidine, "G" denotes deoxyguanosine, and "T" denotes thymidine, unless otherwise noted. Analogs of phosphodiester linkages include phosphorothioate, phosphorodithioate, phosphoranilidate, phosphoramidate, and the like. Generally, oligonucleotide probes of the invention will have a sufficient number of phosphodiester linkages adjacent to its 5' end so that the 5' fwdarw 3' exonuclease activity employed can efficiently degrade the bound probe to separate the reporter and quencher molecules.

"Perfectly matched" in reference to a duplex means that the poly- or oligonucleotide strands making up the duplex form a double stranded structure with one other such that every nucleotide ineach strand undergoes Watson-Crick base pairing with a nucleotide in the other strand. The term also comprehends the pairing of nucleoside analogs, such as deoxyinosine, nucleosides with 2-aminopurine bases, and the like, that may be employed. Conversely, a "mismatch" in a duplex between a target polynucleotide and an oligonucleotide probe or primer means that a pair of nucleotides in the duplex fails to undergo Watson-Crick bonding.

Oligonucleotide probes of the invention can be synthesized by a number of approaches, e.g. Ozaki et at, Nucleic Acids Research, 20:5205-5214 (1992); Agrawal et at, Nucleic Acids Research, 18:5419-5423 (1990); or the like. The oligonucleotide probes of the invention are conveniently synthesized on an automated DNA synthesizer, e.g. an Applied Biosystems, Inc. Foster City, Calif.) model 392 or 394 DNA/RNA Synthesizer, using standard chemistries, such as phosphoramidite chemistry, e.g. disclosed in the following references: Beaucage and Iyer, Tetrahedron, 48:2223-2311 (1992); Molko et al, U.S. Pat. Nos. 4,980,460; Koster et al, U.S. Pat. No. 4,725,677; Caruthers et al, U.S. Pat. Nos. 4,415,732; 4,458,066; and 4,973,679; and the like. Alternative chemistries, e.g. resulting in non-natural backbone groups, such as phosphorothioate, phosphoramidate, and the like, may also be employed provided that the hybridization efficiencies of the resulting oligonucleotides and/or cleavage efficiency of the exonuclease employed are not adversely affected. Preferably, the oligonucleotide probe is in the range of 15-60 nucleotides in length. More preferably, the oligonucleotide probe is in the range of 18-30 nucleotides in length. The precise sequence and length of an oligonucleotide probe of the invention depends in part on the nature of the target polynucleotide to which it binds. The binding location and length may be varied to achieve appropriate annealing and melting properties for a particular embodiment. Preferably, the 3' terminal nucleotide of the oligonucleotide probe is blocked or rendered incapable of extension by a nucleic acid polymerase. Such blocking is conveniently carried out by the attachment of a reporter or quencher molecule to the terminal 3' carbon of the oligonucleotide probe by a linking moiety. Preferably, reporter molecules are fluorescent organic dyesderivatized for attachment to the terminal 3' carbon or terminal 5' carbon of the probe via a linking moiety. Preferably, quencher molecules are also organic dyes, which may or may not be fluorescent, depending on the embodiment of the invention. For example, in a preferred embodiment of the invention, the quencher molecule is fluorescent. Generally, whether the quencher molecule is fluorescent or simply releases the transferred energy from the reporter by non-radiative decay, the absorption band of the quencher should substantially overlap the fluorescent emission band of the reporter molecule. Non-fluorescent quencher molecules that absorb energy from excited reporter molecules, but which do not release the energy radiatively, are referred to herein as chromogenic molecules. There is a great deal of practical guidance available in the literature for selecting appropriate reporter-quencher pairs for particular probes, as exemplified by the following references: Clegg "Fluorescence resonance energy transfer and nucleic acids," Methods of Enzymology, 211: 353-389 (1992), Wu et al; "Resonance energy transfer: methods and applications," Anal. Biochem. 218: 1-13 (1994).; Pesce et at, editors, Fluorescence Spectroscopy (Marcel Dekker, New York, 1971); White et at, Fluorescence Analysis: A Practical Approach (Marcel Dekker, New York, 1970); and the like. The literature also includes references providing exhaustive lists of fluorescent and chromogenic molecules and their relevant optical properties for choosing reporter-quencher pairs, e.g. Berlman, Handbook of Fluorescence Sprectra of Aromatic Molecules, 2nd Edition (Academic Press, New York, 1971 ); Griffiths, Colour and Consitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Fluorescence and Phosphorescence (Interscience Publishers, New York, 1949); and the like. Further, there is extensive guidance in the literature for derivatizing reporter and quencher molecules for covalent attachment via common reactive groups that can be added to an oligonucleotide, as exemplified by the following references: Haugland (cited above); Ullman et al, U.S. Pat. No. 3,996,345; Khanna et al, U.S. Pat. No. 4,351,760; and the like.

Exemplary reporter-quencher pairs may be selected from xanthene dyes, including fluoresceins, and rhodamine dyes. Many suitable forms of these compounds are widely available commercially with substituents on their phenyl moieties which can be used as the site for bonding or as the bonding functionality for attachment to an oligonucleotide. Another group of fluorescent compounds are the naphthylamines, having an amino group in the alpha or beta position. Included among such naphthylamino compounds are 1-dimethylaminonaphthyl-5-sulfonate, 1-anilino-8-naphthalene sulfonate and 2-p-touidinyl-6-naphthalene sulfonate. Other dyes include 3-phenyl-7-isocyanatocoumarin, acridines, such as 9-isothiocyanatoacridine and acridine orange; N-(p-(2- benzoxazolyl)phenyl)maleimide; benzoxadiazoles, stilbenes, pyrenes, and the like. Preferably, reporter and quencher molecules are selected from fluorescein and rhodamine dyes. These dyes and appropriate linking methodologies for attachment to oligonucleotides are described in many references, e.g. Khanna et al (cited above); Marshall, Histochemical J., 7:299-303 (1975); Mechnen et at, U.S. Pat. No. 5,188,934; Menchen et al, European pat. No. application 87310256.0; and Bergot et al, International application PCT/US90/05565. The latter four documents are hereby incorporated by reference.

There are many linking moieties and methodologies for attaching reporter or quencher molecules to the 5' or 3' termini of oligonucleotides, as exemplified by the following references: Eckstein, editor, Oligonucleotides and Analogues: A Practical Approach (IRL Press, Oxford, 1991 ); Zuckerman et al, Nucleic Acids Research, 15: 5305-5321 (1987)(3' thiol group on oligonucleotide); Sharma et al, Nucleic Acids Research, 19:3019 (1991 )(3' sulfhydryl); Giusti et al, PCR Methods and Applications, 2:223-227 (1993) and Fung et al, U.S. Pat. No. 4,757,141 (5' phosphoamino group via Aminolink™. II available from Applied Biosystems, Foster City, Calif.); Stabinsky, U.S. Pat. No. 4,739,044 (3' aminoalkylphosphoryl group); Agrawal et al, Tetrahedron Letters, 31:1543-1546 (1990)(attachment via phosphoramidate linkages); Sproat et al, Nucleic Acids Research, 15:4837 (1987)(5' mercapto group); Nelson et al, Nucleic Acids Research, 17:7187-7194 (1989)(3' amino group); and the like. Preferably, commercially available linking moieties are employed that can be attached to an oligonucleotide during synthesis, e.g. available from Clontech Laboratories (Palo Alto, Calif.).

Rhodamine and fluorescein dyes are also conveniently attached to the 5' hydroxyl of an oligonucleotide at the conclusion of solid phase synthesis by way of dyes derivatized with a phosphoramidite moiety, e.g. Woo et al, U.S. Pat. No. 5,231,191; and Hobbs, Jr. U.S. Pat. No. 4,997,928.

The selection of primers and probes used in this invention is at the choice of the practitioner. This invention places no unique requirements or restrictions on primer or probe choice. Such choices are within the skill of the art.

The probes can be of any length, so long as that length allows one to practice the invention. As a baseline the longer probe must have at least 3 base pairs; as a practical matter the longer probe will be comprised of more than 3 base pairs. However, there is no upper limit occasioned by the use of FETCH. The length of the longer probe is not dictated by the application of FETCH as it will work with any length of probe, over and above the basic requirement of being at least 3 base pairs long. As a practical matter the longer probe will have a length which insures that it hybridizes uniquely with the target polynucleotide and the shorter probe. The shorter probe will be at least 2 fewer base pairs than that of the longer probe. This is the basic standard for creating the shorter probe. It has been found that a good fit as regards the difference in length between the probes can be arrived at by calculating the dissociation temperature of the annealed probes. As a general rule the dissociation temperature of the primers needs to be higher than about 55 degrees C and lower than 90 degrees C. A convenient means for doing this calculation is to use the software called Gene Runer (Hastings Software, Inc.), for example version 3.04.

The shorter gene may be prepared as a 5' truncate of the longer gene. Or it may be a 3' truncate. A third option is to create the shorter probe by truncating both the 5' and the 3' end of the longer gene. Any one of these three forms of the shorter probe will work. While two or more truncated forms could be used, it is simplest to use just one form, preferably the 5' truncate form.

The fluorophore and the quencher can be located on any combination of base pairs so long as the fluorescence of the fluorophore is effectively quenched by the quencher when the two probes are hybridized. The simplest approach is to put the fluorophore on the 5' terminal nucleotide of the longer probe and the quencher on the 3' terminal nucleotide of the shorter probe. This approach can optimizes the quencher molecules affect on the fluorescence of the fluorophore. Preferably, fluorphore and quencher molecules are attached to the terminal 5' carbon and terminal 3' carbon of the probe by way of 5' and 3' linking moieties. However it has been demonstrated that, with at least a number of the fluorophores useful herein, that the fluorophore and the quencher can be situated remotely and still be operative. See for example U.S. patent 5,538,848 which discloses that a fluorophore and quencher may be separated by several nucleotides. That patent discloses work where a fluorophore and quencher are separated by at least 15 nucleotides, or more, and allegedly demonstrate utility. Likewise herein the fluorophore and quencher are permitted to be separated by numerous nucleotides, so long as that separation does not materially reduce the ability of the quencher to affect the signal of the fluorophore when the two probes are hybridized. Of course the fluorophore and/or quencher could be bonded to a nucleotide in the interior of the probes. But for reasons of ease of synthesis and for optimizing hybridization of probe to target and probe to probe, it is preferable to put the fluorophore and quencher on the 5' and 3' terminal ends of the probes, as noted above. In fact the fluorophore and quencher both be on the 5' or 3' terminal nucleotides of their respective oligonucleotides, and the assay will be functional. An alternative arrangement would be to put the fluorophore on the shorter probe and the quencher on the longer probe. Again in this arrangement the longer probe would preferentially bind to the target and thus be separated sufficiently from the probe with the fluorophore so as not to effectively quench its fluorescence. The preferred construct, however, is to have the fluorophore on the longer probe.

The assay can be optimized by varying the ration of the probe with the quencher and the fluorophore probe. A 1:1 ratio gave good results. But it was found that a 2:1 ration of quencher probe to fluorophore probe gave better results. While this aspect of the invention has not been optimized, having been found, one skilled in the art will be able to optimize a given assay by routine testing of different ratios.

The following examples are given to illustrate the invention and are not intended nor should they be read to limit the scope of the invention as claimed in any fashion.

### Examples

### General Description

The use of fluorescence energy transfer by competitive hybridization (FETCH) was developed as an assay for the detection by PCR of hepatitis C virus (HCV) in human specimens. The forward probe, the longer probe, had a 6-FAM dye (carboxyfluoroscein) at the 5' position and a phosphate group at the 3' position (to prevent extension during PCR). The reverse probe, the shorter probe, had a TAMRA dye (N,N,N',N' tetramethyl-6-carboxyrhodamine) at the 3' position (no 3' OH thus no extension possible during PCR). FAM, a commonly used fluorescent dye, was used as the fluorophore. TAMRA, a fluorescent dye with absorption band overlapping the emission band of FAM, was used as the quencher in this application

### Example 1

### Selection of Primers and Probes

HCV polynucleotide sequences were identified from the literature. In order to have an assay which could catch all known HCV strains, two primers and two probes were selected which had polynucleotide sequences common to all reported strain RNA sequences. The primers were selected so that they would anneal to all the known HCV strains and possess satisfactory characteristics such as similar dissociation temperature, no extensive 3' complementarity to each other, etc. The ones chosen flank a 254 bp segment of the 5' non-coding region of HCV from nucleotide number 3290 to 3543 as described in the literature. The HCV sequence data relied on, the primers, and the selected probes are identified in the following Tables I - II and IV (attached).

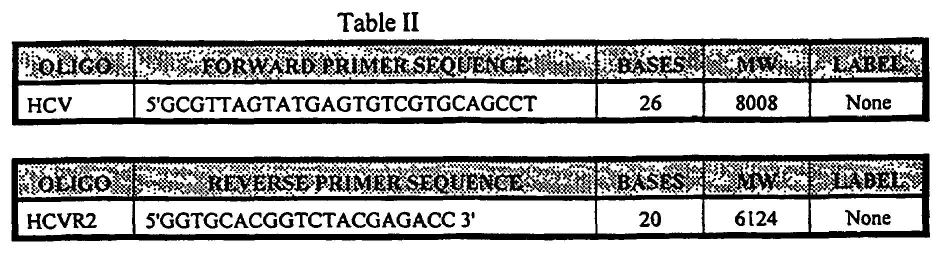

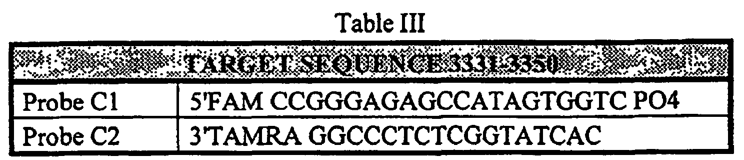

The unconventional presentation of the sequence of C2 (from 3' to 5') is to aid the visualization of its complementarity to C1.

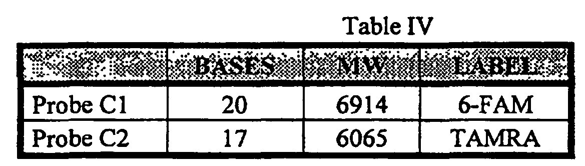

Molecular weighs calculated by Gene Runer version 3.04 (Hastings Software, Inc.)

### Example 2

### Synthesis of Probes

Two probes were custom-synthesized at TriLink Biotechnologies, Inc. (11585 Sorrento Valley Rd., Suite 105, San Diego, CA 92121) They were prepared as follows:
**Step 1. Synthesis of Oligonucleotide**
   The oligonucleotide was prepared on a support that will yield a 3' phosphate group upon deprotection (Glen Research Catalog No. 20-2913).
**Step 2. Addition of 6-FAM**
   The support bound oligonucleotide was then reacted with 15 eqs. of 6-FAM amidite (Glen Research Catalog No. 10-5901) manually to ensure high efficiency.
**Step 3. Deprotection of Oligonucleotide**
   The FAM labeled oligo was deprotected for 36 hours at room temperature with fresh conc. ammonium hydroxide. After deprotection the reagent was decanted, the beads rinsed, and the combined solutions dried.
**Step 4. Purification**
   The FAM labeled oligonucleotide was purified using reverse phase HPLC. The FAM was only on full length material, and was a useful lipophilic handle allowing good separation. After purification, the compound was dried down in preparation for precipitation. The compound was precipitated from 0.3 M NaOAc using EtOH. The product was recovered by high speed centrifugation and washed twice with EtOH.
**Step 5. Final Analysis**
   The dried product was resuspended in water, quantitated, and analyzed by HPLC for purity. The compound was then dried again in preparation for delivery.

### Example 3

### Detection of HCV RNA by Reverse Transcriptase-PCR Using FETCH

The assay was carried out as a one-step reverse transcription and polymerase chain reaction. HCV RNA was isolated and purified from human serum or plasma. The serum or plasma sample was lysed under highly denaturing conditions to inactivate RNAases and to insure isolation of intact RNA. The RNA was precipitated with ethanol and transferred to a QIAmp spin column (Qiagen, Chatsworth, CA) that binds RNA. The column was then washed and RNA eluted with water. The purified RNA template (10 µL) was mixed with HCV master mix (see Table V) (40 µL) and then reverse transcribed to DNA, amplified by PCR and detected in the same tube in the Perkin Elmer 7700 sequence analyzer as per Table VI. During PCR, some of the FAM labeled probe and some of the TAMRA labeled probe annealed to the PCR product thus reducing the quenching of FAM fluorescence and allowed increased fluorescence to be detected. The fluorescence of FAM increase with increasing number of cycles of thermocycling, corresponding with increases in amount of PCR product, as illustrated in Figure 1.

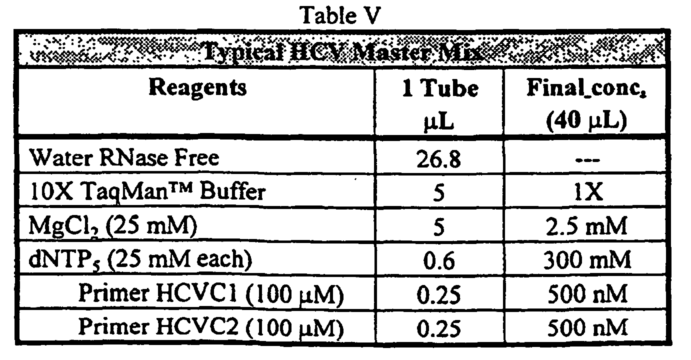

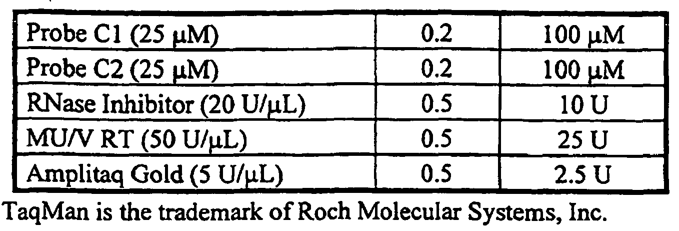

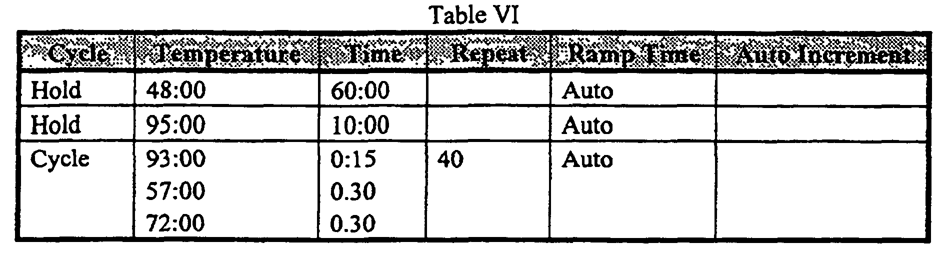

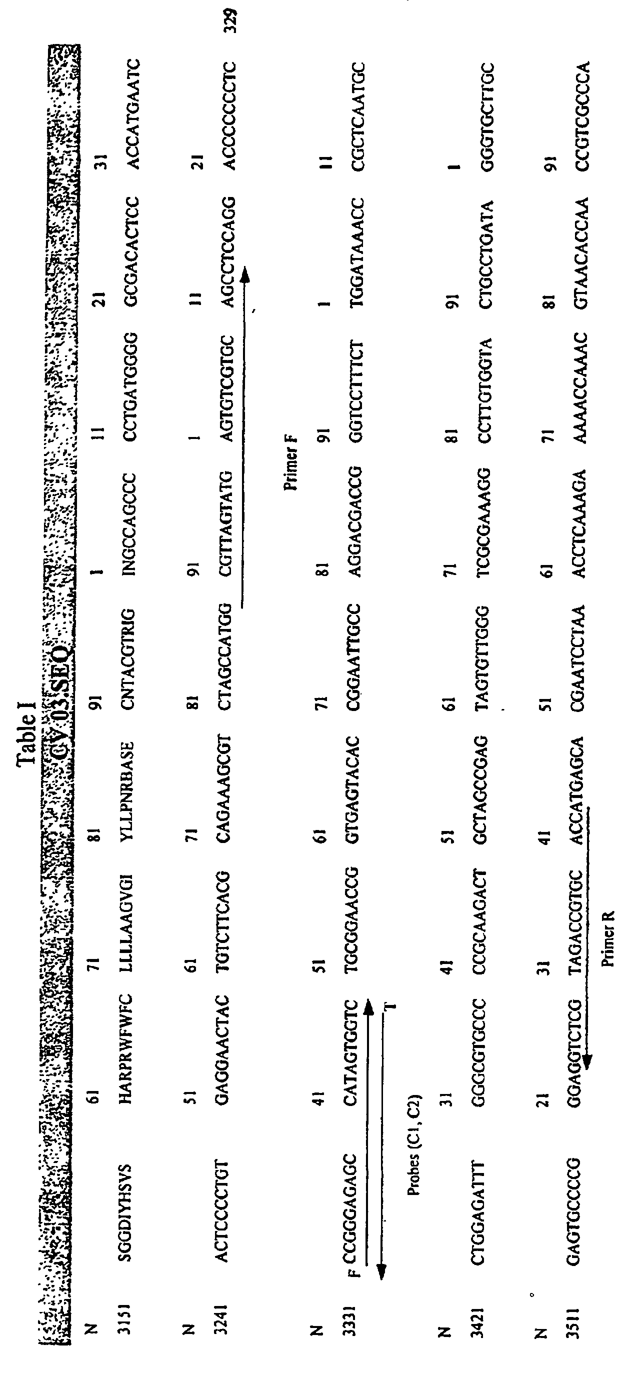

## Claims

1. A method for monitoring nucleic acid amplification comprising:
performing nucleic acid amplification on a target polynucleotide using any method, wherein said amplification is carried out in the presence of a first oligonucleotide probe and a second shorter oligonucleotide probe varying in length by at least 2 base pairs, which allow monitoring of nucleic acid amplification;
the first probe having a fluorophore;
the second being complementary with the first probe and having a quencher molecule capable of quenching the fluorescence of said fluorophore, the fluorophore and quencher being attached on their respective probes at positions which results in the quencher molecule quenching the fluorescence of the fluorophore when the probes are hybridized to each other,
wherein the longer probe binds preferentially to the target polynucleotide and when preferentially bound to the target polynucleotide the fluorescence intensity of the fluorophore is greater than the fluorescence intensity of the fluorophore when hybridized to the second probe, and
monitoring the fluorescence of the fluorophore, the generation of fluorescence corresponding to the occurrence of nucleic acid amplification.

2. The method of claim 1, wherein the nucleic acid polymerase is a thermostable nucleic acid polymerase.

3. The method of claim 1, wherein the fluorophore on the first probe and the quencher molecule on the second probe are on the same hybridized base pair.

4. The method of claim 1, wherein the fluorophore and quencher molecules are within 1 to 3 hybridized base pairs of each other.

5. The method of claim 1, wherein the fluorophore and quencher molecules are within 3 or more hybridized base pairs of each other.

6. The method of claim 1, wherein the fluorophore is on the 5' terminal nucleotide of the first probe and the quencher is on the 3' terminal nucleotide of the second probe.

7. The method of claim 1, wherein the fluorophore is on the 3' terminal nucleotide of the first probe and the quencher is on the 5' terminal nucleotide of the second probe.

8. The method of claim 1, wherein the second probe is complementary to the first probe shortened by deletion of 3 or more 5' terminal nucleotides.

9. The method of claim 1, wherein the second probe is complementary to the first probe shortened by deletion of 3 or more 3' terminal nucleotides.

10. The method of claim 1, wherein the second probe is complementary to the first probe shortened by deletion of 3 or more 5' terminal nucleotides, and deletion of 1 or more 3' terminal nucleotides.

11. The method of claim 1, wherein the first and second probes have a dissociation temperature difference of 2 degrees or more.

12. A method for detecting the presence of specific nucleic acid sequences in a prepared nucleic acid sample comprising:
placing a sample of nucleic acids in a suitable solution and incubating with a first oligonucleotide probe and a second shorter oligonucleotide probe varying in length by at least 2 base pairs;
the first probe having a fluorophore;
the second being complementary with the first probe and having a quencher molecule capable of quenching the fluorescence of said fluorophore, the fluorophore and quencher being attached on their respective probes at positions which results in the quencher molecule quenching the fluorescence of the fluorophore when the probes are hybridized,
wherein the longer probe binds preferentially to the target polynucleotide and when preferentially bound to the target polynucleotide the fluorescence intensity of the fluorophore is greater than the fluorescence intensity of the fluorophore when hybridized to the second probe, and
monitoring the fluorescence of the fluorphore, the generation of fluorescence corresponding to the presence of specific nucleic acid sequences.

## Patentansprüche

1. Verfahren zur Überwachung der Nukleinsäureamplifikation, das umfasst:
- Durchführen einer Nukleinsäureamplifikation eines Targetpolynukleotids mit Hilfe eines beliebigen Verfahrens, wobei die Amplifikation in der Gegenwart einer ersten Oligonukleotidsonde und einer zweiten, kürzeren Oligonukleotidsonde, die in ihrer Länge um mindestens 2 Basenpaare variieren, durchgeführt wird, was die Überwachung der Nukleinsäureamplifikation ermöglicht;
- die erste Sonde, die einen Fluorophor aufweist;
- die zweite Sonde, die zur ersten Sonde komplementär ist, und die ein Quencher-Molekül aufweist, das zum Quenchen der Fluoreszenz des Fluorophors in der Lage ist, wobei der Fluorphor und der Quencher an ihre entsprechenden Sonden an Positionen angeheftet sind, was in dem Quencher-Molekül ein Quenchen der Fluoreszenz des Fluorophors zur Folge hat, wenn die Sonden aneinander hybridisiert sind;
- wobei die längere Sonde vorzugsweise an das Targetpolynukleotid bindet, und wobei, wenn sie das Targetpolynukleotid vorzugsweise gebunden hat, die Fluoreszenzintensität des Fluorophors größer ist als die Fluoreszenzintensität des Fluorophors, wenn sie an die zweite Sonde hybridisiert ist; und
- Überwachen der Fluoreszenz des Fluorophors, wobei die Erzeugung von Fluoreszenz mit dem Auftreten einer Nukleinsäureamplifikation korrespondiert.

2. Verfahren gemäß Anspruch 1, wobei die Nukleinsäurepolymerase eine thermostabile Nukleinsäurepolymerase ist.

3. Verfahren gemäß Anspruch 1, wobei der Fluorophor auf der ersten Sonde und das Quencher-Molekül auf der zweiten Sonde am selben hybridisierten Basenpaar liegen.

4. Verfahren gemäß Anspruch 1, wobei der Fluorophor und Quencher-Moleküle innerhalb von 1 bis 3 hybridisierten Basenpaaren voneinander entfernt liegen.

5. Verfahren gemäß Anspruch 1, wobei der Fluorophor und Quencher-Moleküle innerhalb von 3 oder mehr hybridisierten Basenpaaren voneinander entfernt liegen.

6. Verfahren gemäß Anspruch 1, wobei der Fluorophor am 5'-terminalen Nukleotid der ersten Sonde liegt, und der Quencher am 3'-terminalen Nukleotid der zweiten Sonde liegt.

7. Verfahren gemäß Anspruch 1, wobei der Fluorophor am 3'-terminalen Nukleotid der ersten Sonde liegt, und der Quencher am 5'-terminalen Nukleotid der zweiten Sonde liegt.

8. Verfahren gemäß Anspruch 1, wobei die zweite Sonde komplementär zur ersten Sonde ist, verkürzt durch Deletion von 3 oder mehr 5'-terminalen Nukleotiden.

9. Verfahren gemäß Anspruch 1, wobei die zweite Sonde komplementär zur ersten Sonde ist, verkürzt durch Deletion von 3 oder mehr 3'-terminalen Nukleotiden.

10. Verfahren gemäß Anspruch 1, wobei die zweite Sonde komplementär zur ersten Sonde ist, verkürzt durch Deletion von 3 oder mehr 5'-terminalen Nukleotiden und Deletion von 1 oder mehr 3'-terminalen Nukleotiden.

11. Verfahren gemäß Anspruch 1, wobei die erste und die zweite Sonde einen Unterschied in der Dissoziationstemperatur von 2 Grad oder mehr aufweisen.

12. Verfahren zur Detektion der Gegenwart spezifischer Nukleinsäuresequenzen in einer vorbereiteten Nukleinsäureprobe, das umfasst:
- Einbringen einer Probe aus Nukleinsäuren in eine geeignete Lösung und Inkubieren mit einer ersten Oligonukleotidsonde und einer zweiten, kürzeren Oligonukleotidsonde, die in ihrer Länge um mindestens 2 Basenpaare variieren;
- die erste Sonde, die einen Fluorophor aufweist;
- die zweite Sonde, die zur ersten Sonde komplementär ist, und die ein Quencher-Molekül aufweist, das zum Quenchen der Fluoreszenz des Fluorophors in der Lage ist, wobei der Fluorophor und der Quencher an ihre entsprechenden Sonden an Positionen angeheftet sind, was in dem Quencher-Molekül ein Quenchen der Fluoreszenz des Fluorophors zur Folge hat, wenn die Sonden aneinander hybridisiert sind;
- wobei die längere Sonde vorzugsweise an das Targetpolynukleotid bindet, und wobei, wenn sie das Targetpolynukleotid vorzugsweise gebunden hat, die Fluoreszenzintensität des Fluorophors größer ist als die Fluoreszenzintensität des Fluorophors, wenn sie an die zweite Sonde hybridisiert ist; und
- Überwachen der Fluoreszenz des Fluorophors, wobei die Erzeugung von Fluoreszenz mit der Gegenwart spezifischer Nukleinsäuresequenzen korrespondiert.

## Revendications

1. Procédé de contrôle de l'amplification d'acides nucléiques comprenant :
la réalisation de l'amplification d'acides nucléiques sur un polynucléotide cible en utilisant n'importe quel procédé, où ladite amplification est effectuée en présence d'une première sonde oligonucléotidique et d'une seconde sonde oligonucléotidique plus courte variant en longueur d'au moins 2 paires de bases, qui permettent de contrôler l'amplification d'acides nucléiques;
la première sonde ayant un fluorophore;
la seconde sonde étant complémentaire de la première sonde et ayant une molécule d'extincteur capable d'extinction de la fluorescence dudit fluorophore, le fluorophore et l'extincteur étant fixés sur leurs sondes respectives dans des positions qui ont pour résultat l'extinction par la molécule d'extincteur de la fluorescence du fluorophore lorsque les sondes sont hybridées l'une avec l'autre,
dans lequel la sonde la plus longue se lie de préférence au polynucléotide cible et, lorsqu'elle est liée de préférence au polynucléotide cible, l'intensité de fluorescence du fluorophore est supérieure à l'intensité de fluorescence du fluorophore lorsqu'elle est hybridée avec la seconde sonde, et
le contrôle de la fluorescence du fluorophore, la génération de fluorescence correspondant à l'occurrence de l'amplification d'acides nucléiques.

2. Procédé selon la revendication 1, dans lequel la polymérase d'acide nucléique est une polymérase d'acide nucléique thermostable.

3. Procédé selon la revendication 1, dans lequel le fluorophore sur la première sonde et la molécule d'extincteur sur la seconde sonde sont sur la même paire de bases hybridées.

4. Procédé selon la revendication 1, dans lequel les molécules de fluorophore et d'extincteur sont situées à 1 à 3 paires de bases hybridées l'une de l'autre.

5. Procédé selon la revendication 1, dans lequel les molécules de fluorophore et d'extincteur sont situées à 3 paires ou plus de bases hybridées l'une de l'autre.

6. Procédé selon la revendication 1, dans lequel le fluorophore est situé sur le nucléotide terminal en 5' de la première sonde et l'extincteur est situé sur le nucléotide terminal 3' de la seconde sonde.

7. Procédé selon la revendication 1, dans lequel le fluorophore est situé sur le nucléotide terminal 3' de la première sonde et l'extincteur est situé sur le nucléotide terminal 5' de la seconde sonde.

8. Procédé selon la revendication 1, dans lequel la seconde sonde est complémentaire de la première sonde raccourcie par délétion de 3 nucléotides terminaux en 5' ou plus.

9. Procédé selon la revendication 1, dans lequel la seconde sonde est complémentaire de la première sonde raccourcie par délétion de 3 nucléotides terminaux en 3' ou plus.

10. Procédé selon la revendication 1, dans lequel la seconde sonde est complémentaire de la première sonde raccourcie par délétion de 3 nucléotides terminaux en 5' ou plus et par délétion de 1 nucléotide terminal en 3' ou plus.

11. Procédé selon la revendication 1, dans lequel les première et seconde sondes ont une différence de températures de dissociation de 2 degrés ou plus.

12. Procédé de détection de la présence de séquences spécifiques d'acides nucléiques dans un échantillon préparé d'acides nucléiques, comprenant :
le placement d'un échantillon d'acides nucléiques dans une solution appropriée et l'incubation avec une première sonde oligonucléotidique et une seconde sonde oligonucléotidique plus courte variant en longueur d'au moins 2 paires de bases;
la première sonde ayant un fluorophore;
la seconde sonde étant complémentaire de la première sonde et ayant une molécule d'extincteur capable d'extinction de la fluorescence dudit fluorophore, le fluorophore et l'extincteur étant fixés sur leurs sondes respectives dans des positions qui ont pour résultat l'extinction par la molécule d'extincteur de la fluorescence du fluorophore lorsque les sondes sont hybridées,
dans lequel la sonde la plus longue se lie de préférence au polynucléotide cible et, lorsqu'il est lié de préférence au polynucléotide cible, l'intensité de fluorescence du fluorophore est supérieure à l'intensité de fluorescence du fluorophore lorsqu'elle est hybridée avec la seconde sonde, et
le contrôle de la fluorescence du fluorophore, la génération de fluorescence correspondant à la présence de séquences spécifiques d'acides nucléiques.
